Europäisches Patentamt

European Patent Office

Office européen des brevets

(11) Publication number: **0 383 918**
**A1**

# EUROPEAN PATENT APPLICATION
## published in accordance with Art. 158(3) EPC

(21) Application number: 89901150.6

(22) Date of filing: 26.08.88

(86) International application number:
PCT/SU88/00167

(87) International publication number:
WO 90/01993 (08.03.90 90/06)

(51) Int. Cl.⁵: **B03C 3/68, B03C 3/38**

(43) Date of publication of application:
29.08.90 Bulletin 90/35

(84) Designated Contracting States:
**CH DE FR GB LI SE**

(71) Applicant: **GOSUDARSTVENNY**
**NAUCHNO-ISSLEDOVATELSKY**
**ENERGETICHESKY INSTITUT IMENI**
**G.M.KRZHIZHANOVSKOGO**
**Leninsky pr., 19**
**Moscow, 117921(SU)**

(72) Inventor: **ZHMUROV, Valery Pavlovich**
**ul. Narodnogo Opolchenia, 25-63**
**Moscow, 123154(SU)**
Inventor: **GRINSHTEIN, Boris Iliich**
**ul. Ostrovityanova, 16-4-134**
**Moscow, 117321(SU)**
Inventor: **SHVARTS, Zinovy Lazarevich**
**1 mikroraion, 14-76 Yaroslavskaya**
**obl. Rostov, 152101(SU)**
Inventor: **NAGORNY, Viktor Vladimirovich**
**ul. Mayakovskogo, 14-132**
**Moskovskaya obl. Khimki, 141100(SU)**
Inventor: **BEZUGLY, Stanislav Leontievich**
**ul. Ivanovskaya, 4-9**
**Zaporozhie, 330041(SU)**
Inventor: **KOTLYAR, Vladimir Petrovich**
**ul. Kiyashko, 38-54**
**Zaporozhie, 330065(SU)**
Inventor: **TIMOSHENKO, Anatoly Lukich ul.**
**Remeslennaya,**
**6-75 Moskovskaya obl.**
**Noginsk, 142403(SU)**

(74) Representative: **Crawford, Andrew Birkby**
**A.A. THORNTON & CO. Northumberland**
**House 303-306 High Holborn**
**London WC1V 7LE(GB)**

(54) **PULSED VOLTAGE SOURCE FOR GAS-CLEANING ELECTROFILTERS.**

(57) A pulsed voltage source for gas-cleaning electrofilters comprises a step-up transformer (5), whose primary winding (4) is connected, through a thyristor regulator (1), to a sinusoidal voltage source. To the controlling inputs (15, 16) of the thyristor regulator (1) is connected the output of a control unit (14) whose input is connected to a transmitter (26) of electric parameters of the gas-cleaning electrofilters. The transmitter (26) is connected to the corona and receiving electrodes (10, 12) of the electrofilter (11). The source comprises a rectifying bridge (7), whose inputs are connected to the secondary winding (6) of the step-up transformer (5), a capacitor (17) the first output of which is connected, through a choke coil (19) connected in series with an electronic switch (20), to the corona electrode (10) of the electrofilter (11) and, through a diode (18), to the first output of the rectifying bridge (7), whereas the second output is connected to the receiving electrode (12) of the electrofilter (11) and to the second output of the rectifying bridge (7). The electronic switch (20) is shunted by an oppositely connected rectifier (21). The output power contact (23) and the controlling input of the electronic switch (20) are connected to a pulse generator (25). The source further comprises a resistor (9) whose first output is connected to the first output of the rectifying bridge (7) and the second output to the corona electrode (10) of the gas-cleaning electrofilter (11).

FIG. 1

EP 0 383 918 A1

# PULSE VOLTAGE SUPPLY FOR ELECTROSTATIC PRECIPITATORS

## Field of the Invention

The invention relates to the field of electric gas cleaning, and more specifically to pulse power supplies for electrostatic precipitators.

## Background of the Invention

Widely known in the art is a pulse power supply for electrostatic precipitators (K.Porle et al. "Reducing consumed power exhaust with the aid of pulse supplies of electric precipitators", International Conference on Electrostatic Precipitation, No. 12-15, Kyoto, Japan, p. 77), comprising a thyristor controller connected to a source of sine voltage, a step-up transformer with the primary thereof connected to a thyristor controller. A voltage sensor if connected to the corona-forming and receiving electrodes, with the sensor output connected to the input of a control unit, the outputs whereof are connected to the thyristor gates in the controller. The secondary winding of the step-up transformer drives the inputs of a rectifier bridge. The first terminal of the capacitor is connected via connected in series electronic key and first choke to the corona-forming electrode of the electrostatic precipitator.The electronic key is shunted by a network of connected in series opposition gate and second choke. The second terminal of the capacitor is connected to the receiving electrode of the electrostatic precipitator. A pulse generator drives the heavy-duty contact and the control input of the electronic key.

The pulse repetition rate from the generator driving the heavy-duty contact and the control input of the electronic key is set individually for each gas cleaning process and depends on the properties of the dust being precipitated. The voltage supply further comprises a resistor with the first lead thereof connected to the output of the bridge rectifier and with the second lead thereof connected to the lead of the first choke connected to the electronic key.

With a cutoff electronic key the voltage across the electrostatic precipitator electrodes is approximately equal to that of corona discharge firing.

The capacitor connected across the bridge rectifier output is charged to a voltage, the value whereof is set by the thyristor controller according to an electric signal arriving from the output of the control unit and dependent on the processes taking place in the electrostatic precipitator. Enabling the electronic key causes an oscillatory process in the "capacitor -- electrostatic precipitator -- choke" network, during which the voltage across the electrostatic precipitator electrodes exceeds the voltage drop across the capacitor for a time interval determined by the electric parameters of the electrostatic precipitator and the choke. This produces a bell-shaped pulse voltage across the electrostatic precipitator. After the electronic key is cut off, the voltage across the electrodes drops off to that of the corona discharge. The bell-shaped supply voltage waveform excludes corona backfire, when handling gas an dust with specific resistivities above $10^{10}$ Ohm.m (high-resistance dust). However, the pulsed character of the supply voltage reduces the average voltage across the electrostatic precipitator electrodes.

As is well known, the average voltage across an electrostatic precipitator determines the efficiency of gas cleaning, so that a lower average voltage results in a lower gas cleaning efficiency and dust particle precipitation on the receiving electrodes. When handling gases with high-resistance dust, the development of corona backfire discharges is the main mechanism reducing the efficiency of dust particle charging and precipitation. A pulsed voltage waveform allows elimination of high-resistance dust particle corona backfiring and intensify the process of charging and precipitation of dust particles on the receiving electrode of the electrostatic precipitator.

When cleaning gas with dust particles of a specific resistivity below $10^9$ Ohm.m (medium-resistance dust), a low average voltage of belt-shaped waveform across the electrostatic precipitator results in a lower speed of charged particle drift toward the receiving electrode and thus, despite the higher particle charge, the efficiency of gas cleaning is impaired.

Disclosure of the Invention

This invention is to provide a pulse voltage supply for electrostatic precipitators, the design configuration whereof would improve the gas cleaning efficiency by increasing the average voltage across the electrostatic precipitator electrodes.

This is achieved by that the pulse voltage supply, comprising a step-up transformer with the primary winding thereof connected via a thyristor controller to a sine voltage source, a control unit with the outputs thereof connected to the thyristor gates in the thyristor controller, a sensor of the electric parameters of the electrostatic precipitator with the input thereof connected to the corona-forming and the receiving electrodes of the electrostatic precipitator and with the output thereof connected to the input of the control unit, a bridge rectifier with the inputs thereof connected to the secondary winding of the step-up transformer, a capacitor with the first lead thereof connected via connected in series choke and electronic key to the corona-forming electrode of the electrostatic precipitator, wherein the electronic key is shunted by an opposition connected gate, and is electrically connected to the first input of the bridge rectifier and with the second

capacitor lead connected to the receiving electrode of the electrostatic precipitator and to the second output of the bridge rectifier, and also a resistor with the first lead thereof connected to the first output of the bridge rectifier, and a pulse generator with the outputs thereof connected to the heavy-duty contact terminal and to the control input of the electronic key, according to this invention further comprises a diode with the cathode thereof connected to the first output of the bridge rectifier and with the anode thereof connected to the first lead of the capacitor, wherein the second lead of the resistor is connected directly to the corona-forming electrode of the electrostatic precipitator.

This invention allows improving the efficiency of gas cleaning from dust with specific resistivity below $10^9$ Ohm.m (medium-resistance dust) by increasing the average voltage across the electrodes the electrostatic precipitator energized by a pulse voltage.

Brief Description of the Accompanying Drawings

These and other objectives and advantages of the invention will become apparent from the following detailed description of the pulse voltage supply with reference to specific embodiments thereof and to the accompanying drawings, wherein:

Fig.1 snows the block diagram of the pulse voltage supply for electrostatic precipitators, according to the invention;

Fig. 2a snows the time dependence of the output voltage of the sine voltage source;

Fig. 2b shows the time dependence of the voltage across the electrostatic precipitator electrodes, according to the invention;

Fig. 2c snows the time dependence of the control unit output voltage;

Fig. 2d shows the time dependence of the pulse generator output voltage.

Preferred Embodiment of the Invention

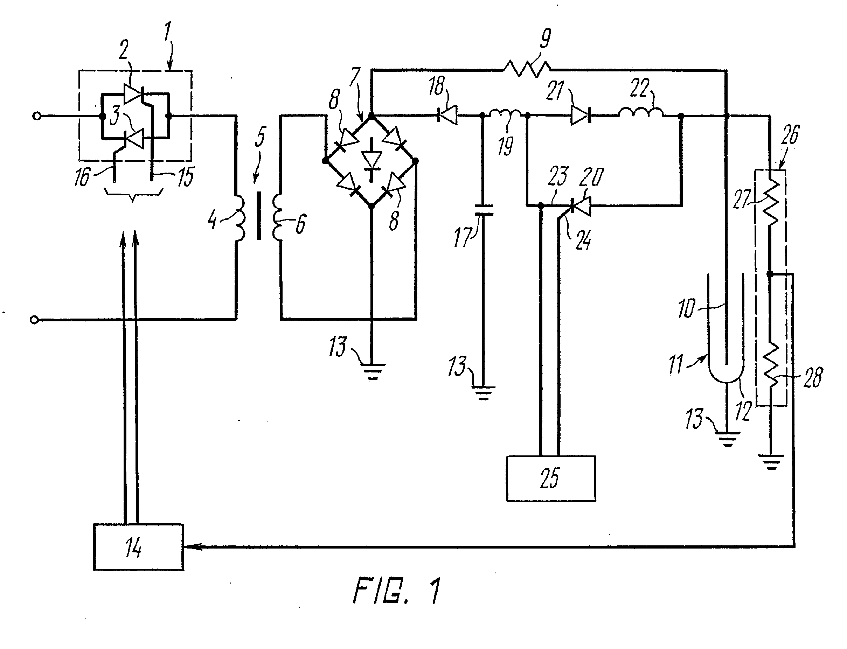

The pulse voltage supply for electrostatic precipitators comprises thyristor controller 1 (Fig. 1) with two thyristors 2, 3 in parallel-opposition connection. Thyristor controller 1 is connected to the first terminal of primary winding 4 of step-up transformer 6. Primary winding 4 has its first terminal connected via thyristor controller 1 and its second terminal connected directly to a sine voltage source (not shown in the drawing). Secondary winding 6 of step-up transformer 5 is connected to the inputs of bridge rectifier 7 comprising diodes 8. The first output of bridge rectifier 7 (the negative pole) is connected via resistor 9 to corona-forming electrode 10 of electrostatic precipitator 11, receiving electrode 12 whereof is connected to common bus 13. The second output of bridge rectifier 7 is(positive pole) connected to common bus 13. The supply also comprises control unit 14 of known in prior art design (T.M.Aliev "Agregati pitaniya elektrofil'trov" ("Electrostatic Precipitator Power Supplies"), Energoizdat Publishing House (Moscow), 1981, pp. 53-60.- In Russian). The output of control unit 14 is connected to gates 15,16 of thyristors 2, 3 in thyristor controller 1. The voltage supply further comprises capacitor 17 with the first lead thereof connected via diode 18 to the first output of bridge rectifier 7.

The negative pole of bridge rectifier 7 is connected to the cathode of diode 18, the anode whereof is connected to the first lead of capacitor 17. The first lead of capacitor 17 is connected via a series network of first choke 19 and electronic key 20, shunted by series-opposition connected gate 21 and second choke 22, to corona-forming electrode 10 of electrostatic precipitator 11. The second lead of capacitor 17 is connected to common bus 13.

The terminal of heavy-duty contact 23 and control input 224 of electronic key 20 are connected to the outputs of pulse generator 25. The repetition rate of generator 25 output pulses is set individually for each gas cleaning process, depending on the properties of the dust being precipitated, and remains approximately constant during the entire cleaning process.

The voltage supply further comprises a sensor of the electric parameters of the electrostatic precipitator, embodied by voltage sensor 26 designed as a voltage divider connected parallel to electrostatic precipitator 11 and comprising resistors 27,28. The first terminal of voltage sensor 26 is connected to corona-forming electrode 10 of electrostatic precipitator 11, the second terminal - to common bus 13, and the sensor output is connected to the input of control unit 14.

The pulse voltage supply for electrostatic precipitators functions as follows.

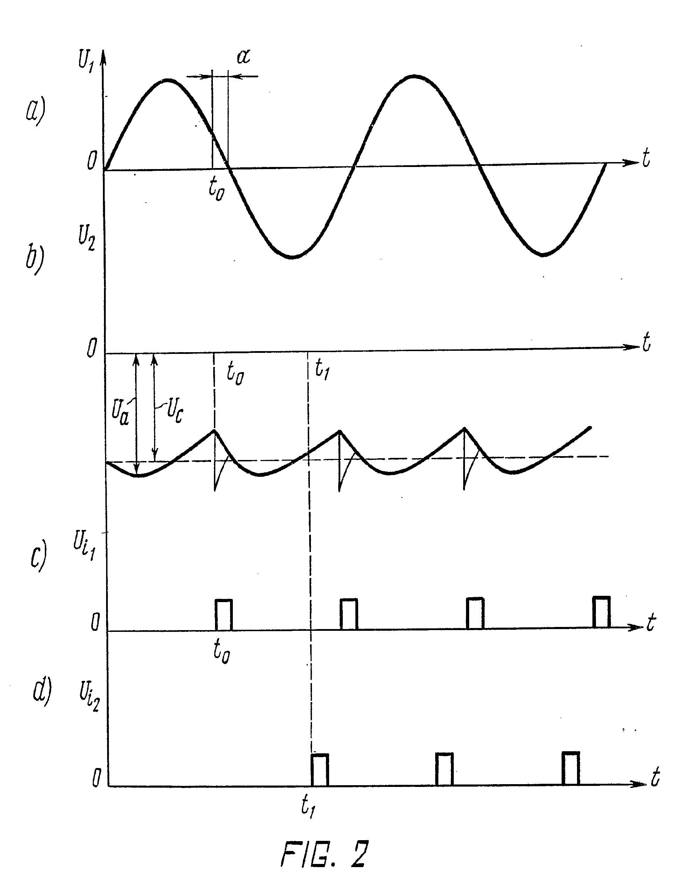

Primary winding 4 (Fig. 1) of step-up transformer 5 via thyristor controller 1 receives voltage $U_1$ (Fig.2a) from the sine voltage source (not shown in the drawings). The voltage from secondary winding 6 of step-up transformer 5 (Fig. 1) is applied to the input of bridge rectifier 7, at the output whereof a voltage is generated with an amplitude and frequency determined by the properties (electric resistance) of the dust precipitated on electrodes 10, 12 of electrostatic precipitator 11. A signal from the output of voltage sensor 26 is passed to the input of control unit 14 and transmits information on the processes taking place in electrostatic precipitator 11, such as the corona discharge in electrostatic precipitator 11, dust particle charge, dust particle drift toward receiving electrode 12 of electrostatic precipitator 11, dust particle precipitation on receiving electrode 12 of electrostatic precipitator 11.

Voltage pulses $U_{i1}$ (Fig.2c) from the output of control unit 14 arrive at gates 15,16 in thyristor controller 1 with a repetition rate algorithm determined by the processes in the electrostatic precipitator 11 (Fig.1), thus setting the firing angle of thyristors 2,3 in thyristor controller 1. As a result, enabling thyristors 2,3 of thyristor controller 1 at the moment of time $t_o$ causes voltage $U_1$ (Fig.2a) from step-up transformer 5 to be rectified by bridge rectifier 7 (Fig. 1) and arrive via resistor 9 at electrodes 10,12 of electrostatic precipitator 11 to charge the equivalent capacitance of the network "corona-forming electrode 10 -- receiving electrode 12" of electrostatic precipitator 11. At a disabled electronic key 20 the voltage across electrodes 10, 12 features that of a rectified voltage with ripple, with an amplitude $U_a$ (Fig.2b) and a ripple frequency equal twice that of voltage $U_1$ (Fig.2a) from the sine voltage source. The maximum ripple voltage in voltage $U_2$ (Fig.2b) is about 1.45.

According to the signal arriving from the output of control unit 14 (Fig. 1), voltage $U_2$ (Fig. 2b) is maintained at a maximum, whereat the interelectrode space of electrostatic precipitator breaks down. At the same time as the equivalent interelectrode capacitance of electrostatic precipitator 11 (Fig.1), capacitor 17 is charged via diode 18 and gate 21 to the amplitude value $U_1$ (Fig.2a).

Under these conditions diode 17 cuts off capacitor 17 from electrostatic precipitator 11 and, during interlectrode break-down in electrostatic precipitator 11 and with a disabled electronic key 20 capacitor 17 is not discharged via resistor 9 onto corona-forming electrode 10 and receiving electrode 12, this preventing overvoltage on these electrodes of electrostatic precipitator 11.

Enabling electronic key 20 (Fig. 1) and applying voltage pulses $U_{12}$ from the output of pulse generator 25 to the terminal of heavy-duty contact 23 and to control input 24 of electronic key 20 at the moment of time $t_1$ (Fig. 2d) causes an oscillatory process in the electric network "capacitor 17 -- electrodes 10,12 of electrostatic precipitator 11". As a result voltage pulses $U_i$ (Fig. 2b) at electrodes 10,12 of electrostatic precipitator 11 are superimposed on rectified voltage $U_2$ with ripple, with pulse durations of dozens, hundreds of microseconds.

Due to the value of resistor 9 being 10 kOhm to 20 kOhm and the losses herein being relatively low, and also due to the leads of resistor 9 being connected directly to the output of bridge rectifier 7 and to corona-forming electrode 10 of electrostatic precipitator, 11 the amplitude value of voltage $U_a$ (Fig. 2b) across the corona-forming and receiving electrodes 10,12 (Fig. 1) of electrostatic precipitator 11 is increased, this leading to a correspondingly higher average voltage $U_c$ (Fig.2b) across electrodes 10,12 of electrostatic precipitator 11.

Using the pulse voltage supply for electrostatic precipitators of this invention allows the efficiency of gas cleaning to be improved, the wear of electrostatic precipitator components to be reduced, the service life of electrostatic precipitators to be prolonged, and their power consumption to be reduced.

. Industrial Applicability

This invention can be used in heat engineering, ferrous and non-ferrous metallurgy, construction material industry.

C L A I M : -

A pulse voltage supply for electrostatic precipitators, comprising step-up transformer (5) with the primary wonding (4) thereof connected via thyristor controller (1) to a source of sine voltage, control unit (47) with the outputs thereof connected to gates (15, 16) of thyristors (2,3) in thyristor controller (1), sensor (26) of the electrostatic precipitator's electric parameters with the inputs thereof connected to corona-forming and receiving electrodes (10, 12) of electrostatic precipitator (11) and with the output thereof connected to the input of control unit (14), bridge rectifier (7) with the inputs thereof connected to secondary winding (6) of step-up transformer (5), capacitor (17) with the first lead thereof connected via connected in series choke (19) and electronic key (20) shunted by opposition connected gate (21) to corona-forming electrode (10) of electrostatic precipitator (11) and electrically connected to the first input of bridge rectifier (7) and with the second lead of capacitor (17) connected to receiving electrode (12) of electrostatic precipitator (11) and to the second output of bridge rectifier (7), and also resistor (9) with the first lead thereof connected to the first output of bridge rectifier (7) and with the second lead thereof electrically connected to corona-forming electrode (10) of electrostatic precipitator (11), and pulse generator (25) with the outputs thereof connected to the terminal of heavy-duty contact (23) and to control input (24) of electronic key (20), c h a r a c t e r i z e d   b y   t h a t   it further comprises diode (18) with the cathode thereof connected to the first output of bridge rectifier (7) and with the anode thereof connected to the first lead of capacitor (17), wherein the second lead of resistor (9) is connected to corona-forming electrode (10) of electrostatic precipitator (11).

Modified Claim for International Patent Application
PCT/SU 88/00167

A pulse voltage supply for electrostatic precipitators, comprising a step-up transformer (5) with primary winding (4) thereof connected via thyristor controller (1) to a source of sine voltage, control unit (14) with the outputs thereof connected to gates (15, 16) of thyristors (2,3) in thyristor controller (1), sensor (26) of the electrostatic precipitator's electric parameters with the inputs thereof connected to the corona-forming and receiving electrodes (10,12) of electrostatic precipitator (11) and with the output thereof connected to the input of control unit (14), bridge rectifier (7) with the inputs thereof connected to secondary winding (6) of step-up transformer (5), capacitor (17) with the first lead thereof connected via connected in series choke (19) and electronic key (20) shunted by opposition connected gate (21) to corona-forming electrode (10) of electrostatic precipitator (11) and electrically connected to the first input of bridge rectifier (7) and with the second lead of capacitor (17) connected to receiving electrode (12) of electrostatic precipitator (11) and to the second output of bridge rectifier (7), and also resistor (9) with the first lead thereof connected to the first output of bridge rectifier (7) and pulse generator (25) with the outputs thereof connected to the terminal of heavy-duty contact (23) and to control input (24) of electronic key (20), c h a r a c - t e r i z e d   b y   t h a t   it further comprises diode (18) with the cathode thereof connected to the first output of bridge rectifier (7) and with the anode thereof connected to the first lead of capacitor (17), wherein the second lead of resistor (9) is connected to corona-forming electrode (10) of electrostatic precipitator (11).

FIG. 1

EP 0 383 918 A1

FIG. 2

# INTERNATIONAL SEARCH REPORT

International Application No PCT/SU 88/00167

## I. CLASSIFICATION OF SUBJECT MATTER (if several classification symbols apply, indicate all) *

According to International Patent Classification (IPC) or to both National Classification and IPC

$IPC^4$ B 03 C 3/68, 3/38

## II. FIELDS SEARCHED

### Minimum Documentation Searched [7]

| Classification System | Classification Symbols |
|---|---|
| $IPC^4$ | B 03 C 3/68, 3/38 |

Documentation Searched other than Minimum Documentation
to the Extent that such Documents are Included in the Fields Searched [8]

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| A | US, A, 4626261, (F.L. SMIDTH & CO. A/S), 02 December 1986 (02.12.86), see fig. 1 | 1 |
| A | DE, A1, 3525557, (SUMITOMO HEAVY INDUSTRIES, LTD.), 30 Janaury 1986 (30.01.86), see figs. 5,13 | 1 |
| A | SU, A1, 1282100, (Nauchno-issledovatelsky i proektny institut po gazoochistnym sooruzheniyam,tekhnike beropasnosti i okhrane truda v promyshlennosti stroitelnykh materialov), 07 January 1987 (07.01.87), see the drawing | 1 |
| A | SU, A1, 1268207, (Moskovskoe otdelenie nauchno-issledovatelskogo instituta po peredache elektroenergii postoyannym tokom vysokogo napryazhenia) 07 November 1986 (07.11.86), see fig. 1 | 1 |

------------------

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 17 May 1989 (17.05.89) | 24 May 1989 (24.05.89) |

| International Searching Authority | Signature of Authorized Officer |
|---|---|
| ISA/SU | |

Form PCT/ISA/210 (second sheet) (January 1985)